Europäisches Patentamt

European Patent Office

Office européen des brevets

(19)

(11) Publication number: **0 288 622 B1**

# (12) EUROPEAN PATENT SPECIFICATION

(45) Date of publication of patent specification: **11.09.91**  (51) Int. Cl.5: **B01J 29/22**, C10G 45/64, C07C 5/22

(21) Application number: **87303740.2**

(22) Date of filing: **28.04.87**

(54) **Process for the isomerization of paraffinic hydrocarbons.**

(43) Date of publication of application:
**02.11.88 Bulletin 88/44**

(45) Publication of the grant of the patent:
**11.09.91 Bulletin 91/37**

(84) Designated Contracting States:
**AT BE CH DE ES FR GB GR IT LI NL SE**

(56) References cited:
**EP-A- 0 049 803**
**FR-A- 2 067 081**
**US-A- 3 507 931**
**US-A- 4 489 216**

(73) Proprietor: **UOP (a New York general partnership)**
**25 East Algonquin Road**
**Des Plaines Illinois 60017-5017(US)**

(72) Inventor: **Johnson, James A.**
**225 Holmes Drive**
**Clarendon Hills Illinois 60514(US)**
Inventor: **Schmidt, Robert J.**
**2805 Pebblebrook**
**Rolling Meadows Illinois 60008(US)**
Inventor: **Bakas, Steve T.**
**10330 South Austin Avenue**
**Chicago Ridge Illinois 60415(US)**
Inventor: **Cole, Steven W.**
**3217 Oak Avenue**
**Brookfield Illinois 60514(US)**

(74) Representative: **Brock, Peter William et al**
**URQUHART-DYKES & LORD 91 Wimpole Street**
**London W1M 8AH(GB)**

## Description

The present invention is related to a hydroisomerization process employing an improved catalytic composition. More particularly, this invention involves a process employing an isomerization catalyst composition comprising a hydrogenation function selected from the Group VIII metals, a hydrogen-form crystalline alumino-silicate zeolite, and a refractory inorganic oxide. The catalytic composite is especially useful for isomerization of sulfur-contaminated isomerizable hydrocarbons.

The isomerization of low molecular weight normal paraffins is well established in the art. This reaction is of considerable importance in the petroleum industry because of the substantially higher octane numbers of isoparaffins compared to their normal paraffin counterparts. Since gasoline blends require a distribution of boiling range materials, the isoparaffins in the $C_4$-$C_7$ range are valuable blending components. It has been the practice up until this time to isomerize paraffins to equilibrium mixtures of their branched chain isomers with a variety of catalysts. Friedel-Crafts catalysts, such as aluminum chloride, are known to be effective isomerization catalysts. Noble metals, such as platinum supported on halogenated alumina or silica-alumina have also been used effectively to isomerize hydrocarbons. More recently, crystalline alumino-silicate zeolites which have shown catalytic activity have been effectively used in the isomerization of hydrocarbons. Both natural and synthetic crystalline alumino-silicates have been employed. Included among these are the Type X and Type Y zeolites as well as synthetic mordenite.

Specifically, the zeolites known as mordenites have received great attention. Mordenites are crystalline natural or synthetic zeolites of the alumino-silicate type: generally, they have a composition expressed in moles of oxide of

$$1.0 \pm 0.2 \; Na_2O \bullet Al_2O_3 \bullet 10 \pm 0.5 \; SiO_2.$$

the quantity of $SiO_2$ may also be larger. Instead of all or part of the sodium, other alkali metals and/or alkaline earth metals may be present.

In general, it has been found that the sodium form of mordenite is not particularly effective for isomerization of hydrocarbons and that replacing all or, for the greater part, the sodium cations with hydrogen ions yields the more advantageous hydrogen form mordenite. Conversion of the sodium form to the hydrogen form can be accomplished by a number of means. One method is the direct replacement of sodium ions with hydrogen ions using an acidified aqueous solution where the process of ion exchange is employed. Another method involves substitution of the sodium ions with ammonium ions followed by decomposition of the ammonium form using a high temperature oxidative treatment.

The activity and selectivity of hydroisomerization catalysts depend on a variety of factors, such as the mode of catalyst preparation, the presence or absence of promotors, quality of raw materials, feedstock quality, process conditions, and the like. Suitable catalysts can be conventionally prepared by combining commercially available crystalline zeolites, such as, a hydrogen-form mordenite, with a suitable matrix material followed by the addition of a Group VIII metal, and thereafter activating by conventional means. A new catalyst has been discovered which exhibits greatly improved isomerization performance when compared to conventionally prepared catalysts.

The process of isomerizing hydrocarbons containing high levels of sulfur can now be effectively accomplished utilizing a catalyst as defined below. As exemplified within the instant application, this catalyst demonstrates superior isomerization performance compared to conventional isomerization catalysts when processing feeds containing sulfur.

Accordingly, there is provided a process for the isomerization of isomerizable hydrocarbons. This process makes use of a catalytic composite which comprises a combination of a platinum group component with a support containing a hydrogen-form alumino-silicate and refractory inorganic oxide, with the essential feature that the composite have a surface area of at least 580 m²/g. Platinum is the preferred a platinum group metal, and is preferably present in the amount of from 0.15 to 0.5 wt. % of said composite. The preferred hydrogen-form alumino-silicate has a silica to alumina ratio of at least 16 and is present in amount from 75 to 95 wt. % of said composite and it most preferably has a mordenite structure. The preferred refractory inorganic oxide is alumina, selected from the group consisting of gamma-alumina, eta-alumina, and mixtures thereof.

In another aspect the invention is a method of manufacturing the aforementioned catalytic composition and a hydroisomerization process employing that catalytic composition. Manufacturing of the catalyst comprises forming a composite comprising hydrogen form mordenite and refractory oxide, thereafter contacting formed composite with an acidic aqueous solution containing ammonium ions, having the composition and under the conditions set out below, followed by incorporation of a platinum group metal into the formed composite.

The preferred isomerization process conditions employing the instant catalytic composite comprise a temperature range from 200° F to 800° F (93 to 427° C), a pressure of from 100 to 1,000 psia (690 to 6895 kPa gage) and a liquid hourly space velocity in the range of 0.25 to 5 hr⁻¹. Hydrogen is also required in the amount ranging from 0.5 to 5 mole $H_2$/mole hydrocarbon feed.

These, as well as other embodiments of the present invention, will become evident from the following, more detailed description.

The prior art recognizes a myriad of catalyst formulations for the isomerization of hydrocarbons. It is well known that acids, such as, strong mineral acids, can be used to modify crystalline alumino-silicate powders through decationization and dealumination. Ammonium compounds have also been successfully employed to convert crystalline alumino-silicates from alkali and/or alkaline metal cation form to the hydrogen-form.

Combinations of the acid and ammonium treatments have been disclosed for use on alumino-silicate powders. U.S. Patent 3,475,345 (Benesi) discloses a method of converting alumino-silicate zeolites, particularly a sodium form synthetic mordenite, to the hydrogen form utilizing a three-step pretreatment performed on the powdered zeolite. These pretreatment steps consist of 1) a hot acid treatment, 2) a cold acid treatment, and 3) treatment with an ammonium compound. U.S. Patent 3,442,794 (Van Helden et al.) also discloses a method for the pretreatment of alumino-silicate zeolites to the hydrogen form. Again, the preferred zeolite is the synthetic sodium form of mordenite. The method disclosed is very similar to U.S. 3,475,345 mentioned above, with the distinguishing feature being a separately performed two-step pretreatment with 1) an acid compound, and 2) an ammonium compound in arbitrary order. An important feature of both references is that the treatments are performed solely on the alumino-silicate zeolite with the express intention of modifying said zeolite before being utilized in a catalyst formulation and that no mention of the importance of the surface area of the catalytic composite is disclosed. This is distinguished from the present invention in that any treatment performed is subsequent to the zeolite being incorporated into a formed catalyst composite and more importantly without any apparent modification of the zeolite itself.

Treatment of the alumino-silicates with acid have not only been effective for conversion to the hydrogen form, but also have been used as a means for increasing the silica to alumina ratio. Typically, a silica to alumina ratio of about 10:1 is observed for a sodium form synthetic mordenite and is substantially unchanged if an ammonium treatment is used to convert the mordenite to the hydrogen form. If a mordenite powder is subjected to an acid treatment as taught in U.S. 3,597,155 (Flanigen) an increase in the silica to alumina ratio is effected. The acid treatment is believed to cause a reduction of the framework tetrahedra aluminum atoms, thus increasing the proportion of silicon atoms present in the zeolite structure. Isomerization performance is enhanced when the silica to alumina ratio of a mordenite powder is increased. As taught in U.S. 3,507,931 (Morris et al), a silica to alumina ratio above about 20:1 significantly improves the isomerization of light hydrocarbons. U.S. 4,018,711 (Bertolacini) also teaches that isomerization performance is improved if a pretreated mordenite powder having a silica to alumina ratio of at least 19:1 is incorporated in a catalytic composition. Again, these references specifically teach the use of acid treatment on the zeolite powder alone for the purpose of increasing the silica to alumina ratio, whereas the subject invention incorporates an already high silica to alumina ratio crystalline alumino-silicate into the catalytic composite. These references also do not teach the importance of the surface area of the catalytic composite or its relationship to isomerization performance.

A common attribute of the above mentioned prior art is that, in all cases, the crystalline alumino-silicate alone, in particular the synthetic sodium form of mordenite, is subjected to an acid and/or an ammonium pretreatment step(s) to modify the alumino-silicate before its incorporation into the catalyst composition. Although the pretreatment of mordenite as described in the above references enhances the isomerization performance of catalytic composites comprising such pretreated mordenite, further improvements are still obtainable.

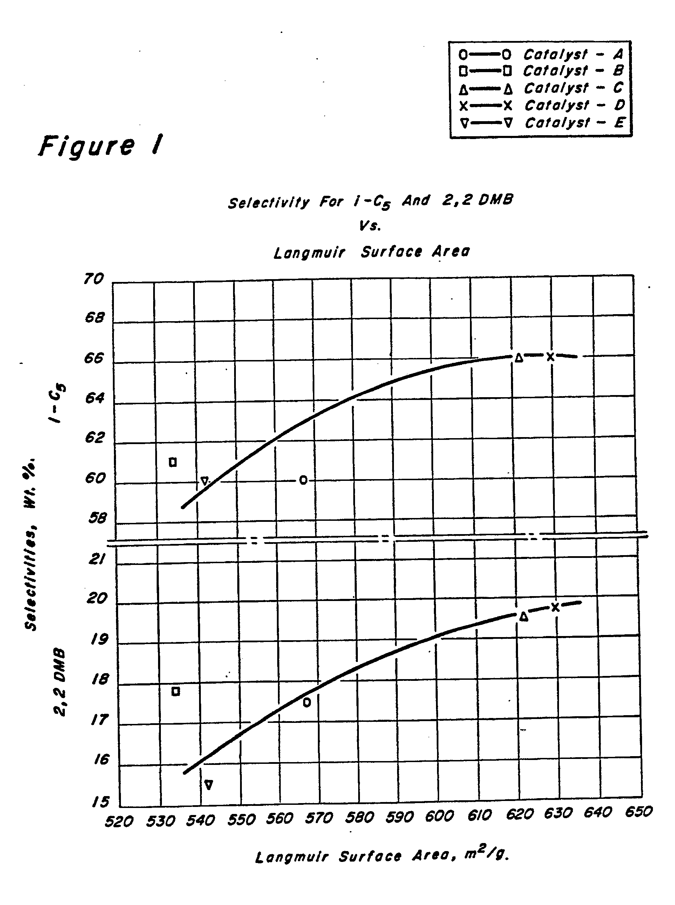

Reference to the accompanying drawings will facilitate understanding of the present invention. The graph shown in Figure 1 of the drawings illustrates that the isomerization performance is a function of the Langmuir surface area of the catalytic composite. The isomerization performance is characterized as selectivity to desired products, namely, 2,2 dimethylbutane (2,2 DMB) and isopentane (i-$C_5$).

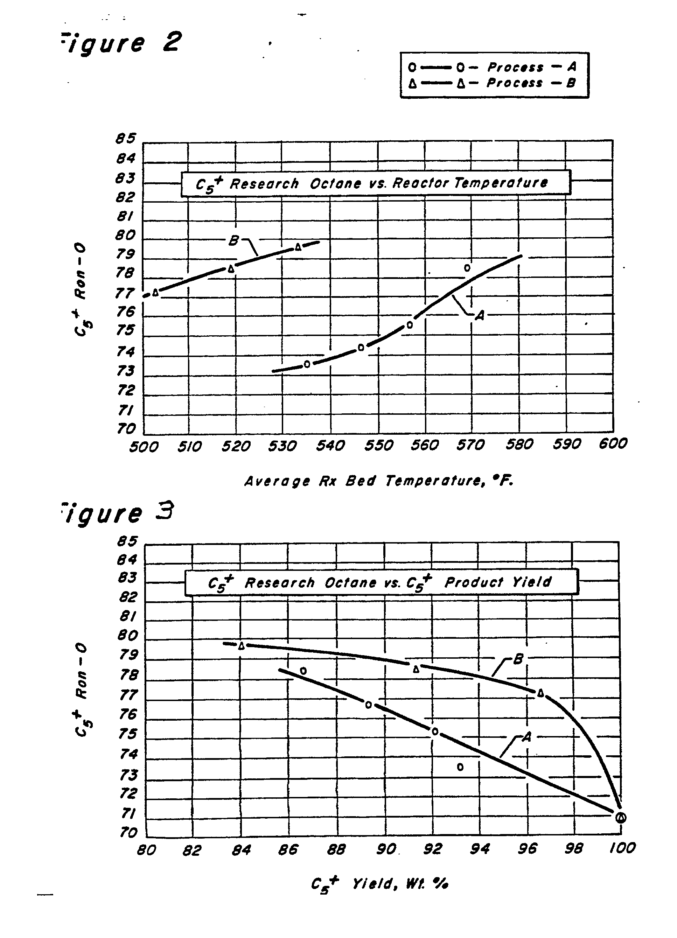

The graph shown in Figure 2 of the drawings illustrates the relationship between the Research octane (RON-O) value of the $C_5$ + isomerization product and the average catalyst bed temperature in the reactor. This RON-O value is also compared in Figure 3 to the $C_5^+$ isomerization product yield, measured as liquid volume percent on a fresh feed basis.

While previous work dealt exclusively with pretreatment of the alumino-silicate component of an isomerization catalyst, it is one of the objects of the present invention to provide a novel catalytic composition which exhibits improved isomerization performance due to carefully controlled treatment of the support.

According to the present invention, there is provided a catalytic composition for the isomerization of isomerizable hydrocarbons into their branched chain equivalents, a method of manufacturing said catalytic composition, and a process which uses said catalytic composition to produce high octane blending components.

The catalyst composition of the present invention comprises a platinum group metal component, hydrogen-form crystalline alumino-silicate, and a refractory inorganic oxide with said catalyst composition having a surface area of at least 580 $m^2/g$. We have found that significant improvements in isomerization performance are realized when the surface area of the catalytic composition is at or above 580 $m^2/g$. Obtaining such a surface area is the object of one of the embodiments of the subject invention and is further illustrated in subsequent examples.

The metal component that is incorporated into the catalytic composite to supply the hydrogenation-dehydrogenation function is a Group VIII noble metal component. The Group VIII noble metals include the metals of the "Platinum Series" and the metals of the "Palladium Series", i.e., platinum, iridium, osmium, palladium, rhodium, and ruthenium which are collectively referred to herein as "platinum group metals." The preferred Group VIII noble metal is platinum. The Group VIII noble metal of the catalytic composition of the present invention will be utilized in an amount from about 0.1 to about 5% by weight of the composite. It is particularly preferred that the metal component be at least about 0.15% by weight and not over 0.5% by weight.

It is within the scope of the instant invention that the catalytic composition also contain a catalytically effective amount of a promoter metal. Examples of such promoter metals include tin, lead, germanium, cobalt, nickel, iron, tungsten, chromium, molybdenum, bismuth, indium, gallium, cadmium, zinc, uranium, copper, silver, gold, tantalum, one or more of the rare earth metals and mixtures thereof.

The crystalline alumino-silicate of the present invention is a hydrogen form silica-alumina having either a three-dimensional or channel-pore-structure crystal lattice framework. The three-dimensional alumino-silicates include both synthetic and naturally occurring silica aluminas, such as, the faujasites which include X-type, Y-type, ultrastable-Y and the like. L-type, omega-type, and mordenite are examples of the channel-pore-structure crystalline alumino-silicates.

The alumino-silicate material that is preferred in the catalytic composition of this invention is the particular form of alumino-silicate material known as mordenite. While mordenite is naturally occurring, a variety of synthetic mordenites are available commercially, usually in a powder form. These synthetic mordenites can be obtained in both the sodium form and hydrogen form and at varied silica to alumina ratios. It is a preferred embodiment of the present invention that the mordenite be of the hydrogen form and that the silica to alumina ratio be at least 16:1, more specifically, in the range from 16:1 to 60:1. The pretreatment steps taught in the aforementioned references are routinely and typically employed in the manufacture of commercial available mordenite powders which meet the requirements as a starting material as set forth in the present invention. These pretreatment steps are used to increase the silica to alumina ratio of the mordenite zeolite and to convert the sodium form to the more desirable hydrogen form.

The hydrogen form alumino-silicate is incorporated with a refractory inorganic oxide and formed into a catalyst composite. The formed catalyst composite may be prepared by any known method in the art including the well-known oil drop and extrusion methods. The hydrogen form alumino-silicate may be present in an amount within the range of 50 to about 99.5 weight percent, preferably within the range of 75 to about 95 weight percent, and the refractory inorganic oxide may be present in an amount within the range of from 0.5 to about 50 percent.

The preferred inorganic oxide for use in the present invention is alumina. The alumina is preferably selected from the group consisting of gamma-alumina, eta-alumina and mixtures thereof. Other refractory inorganic oxides which are contemplated include, for example, silica gel, silica-alumina, magnesia-alumina, zirconia-alumina, and the like.

Surprisingly and unexpectedly, it has been found that a strong correlation exists between the isomerization performance of a catalyst composite of the subject invention and the surface area of said composite. One preferred method to achieve maximum isomerization performance is by utilizing formed catalytic composites with surface areas measuring at least 580 $m^2/g$. Surface area, as referred to herein, is determined by employing the Langmuir method of correlating adsorption/desorption isotherm data. The Langmuir method is especially suitable for catalytic composites containing high percentages of crystalline alumino-silicates. The data needed for the Langmuir method is typically obtained by well known adsorption/desorption apparatuses, preferably a nitrogen adsorption/desorption apparatus. Any method may be employed which results in a final catalyst composite having at least a surface area of 580 $m^2/g$. Catalytic composites with high surface areas can be arrived at in a number of ways, such as, using a crystalline alumino-silicate powder which inherently has a very high surface area, or by having one component of the composite, which has a high surface area, in great proportion to other components. A preferred method of achieving a surface area of at least 580 m²/g is to contact the formed catalyst composite with an acidic aqueous solution containing ammonium ions, as defined below. The formed catalyst composite may be dried and/or calcined prior to its contact with the aqueous solution.

The acidic nature of the aqueous solution is attained by employing an acid. Particularly suitable are strong mineral acids such as $H_3PO_4$, $H_2SO_4$, and $HNO_3$ and HCl. HCl is the preferred acid of the present invention. Of course, it is contemplated that mixtures of various acids may also be employed. The preferred source of the ammonium ion incorporated in the acidic aqueous solution is $NH_4Cl$, but any ammonium compound which can form ammonium ions, such as $NH_4OH$, $NH_4NO_3$, $NH_4$ sulfates, $NH_4$ phosphates and the like, should be suitable.

Concentrations of the acid and ammonium ions in the aqueous solution are from 0.5 M to 6 M for the acid concentration and 0.5 M to 4 M for the ammonium ion concentration. Particularly good results are obtained using a solution containing acid and ammonium ion concentrations within the range of 2 to 5 M for the acid and 1 to 3 for the ammonium ion.

A plurality of methods for contacting the formed catalytic composition and the acidic aqueous solution containing ammonium ions is envisioned with no one method of particular advantage. Such contacting methods may include, for example, a stationary catalyst bed in a static solution, a stationary catalyst bed in an agitated solution, a stationary catalyst bed in a continuously flowing solution or any other means which efficiently contacts the catalyst composition with the acidic aqueous solution.

The temperature of the contacting solution should be within the range of 25°C (77°F) to 100°C (212°F), preferably within the range of from 50°C (122°F) to 98°C (208°F). The time required for the contacting step will depend upon concentrations, temperature and contacting efficiency. In general, the contacting time should be at least 0.5 hour, but not more 4 hours, preferably between 1 and 3 hours in duration.

As a result of contacting the formed catalytic composition with the acidic aqueous solution containing ammonium ions, an increase in the measured surface area is observed. Surprisingly and unexpectedly, this increase in surface area, to 580 m²/g or higher, is not accompanied by an increase in the silica to alumina ratio of the hydrogen form crystalline alumino-silicate as measured by Magic Angle Spinning NMR (MASNMR). The MASNMR technique, which is a well known analytical method of the art, indicates no reduction in the framework tetrahedral aluminum atoms of catalyst compositions of the present invention. As will be demonstrated hereinafter, catalysts of the instant invention with 580 m²/g surface area surprisingly and unexpectingly exhibit improved isomerization performance.

The catalyst of the invention has particular utility for the isomerization of isomerizable hydrocarbons. Included in the group of isomerizable hydrocarbons are saturated hydrocarbons including paraffin hydrocarbons and is still more particularly suitable for the hydroisomerization of straight chain or slightly branched chain paraffins containing four or more carbon atoms per molecule. The isomerization reaction can be conducted over a wide range of temperatures, but, in general, in the range from about 93°C (200°F) to about 427°C (800°F). Space velocities from about 0.25 to about 5 liquid volumes per hour of said isomerizable hydrocarbons per volume of said catalytic composite are preferred with reaction zone pressures preferably within the range from about 6.9 bar (100 psi) to about 69 bar (1000 psi). It is particularly desirable to carry out the isomerization reaction in the presence of hydrogen preferably in the range from about 0.5 to about 5 moles of $H_2$ per mole of isomerizable hydrocarbon. The function of the hydrogen is primarily to improve catalyst life, apparently by preventing polymerization of intermediate reaction products which would otherwise polymerize and deposit on the catalytic composite. It is not necessary to employ pure hydrogen since hydrogen containing gases, e.g., hydrogen-rich gas from the catalytic reforming of naphthas are suitable.

The process of this invention is more specifically applicable to isomerizable hydrocarbons including acyclic paraffins and cyclic naphthenes. It is applicable to straight or partially branched chain paraffins such as normal butane, normal pentane, normal hexane, normal heptane, normal octane, 2 methylpentane, 3 methylpentane, 3 ethylpentane, etc. It is also applicable to cycloparaffins such as alkylcyclopentanes and cyclohexanes, and methyl cyclopentane, dimethyl cyclopentane, cyclohexane, methyl cyclohexane, dimethyl cyclohexane, etc. The present inventive process is also applicable to mixtures of paraffins and/or naphthenes, including those derived from selective fractional distillation of natural gasolines and naphtha. Examples of such mixtures of paraffins and/or naphthenes include the so-called pentane fractions, hexane fractions, and mixtures thereof. This invention is not limited in its application to the enumerated saturated hydrocarbons. It is contemplated that straight or branched chain saturated hydrocarbons containing up to about 20 carbon atoms per molecule may be isomerized within the scope of the present invention. A preferred class of isomerizable hydrocarbons include those hydrocarbons having four to seven carbon atoms per molecule.

Straight-run hydrocarbons applicable to the process of this invention can contain sulfur compounds in an amount ranging from about 1 wt. ppm to about 300 wt. ppm, calculated as elemental sulfur. It is an advantage of the instant invention that the combined feed to the isomerization reaction zone may contain $H_2S$ and/or organic sulfur compounds in an amount to provide an elemental sulfur content from about 5 wt. ppm to about 200 wt. ppm of the combined feed. The term "combined feed" as used herein means an admixture of process streams to be contacted with the isomerization catalyst. These process streams include fresh isomerizable hydrocarbons, recycled isomerizable hydrocarbons, $H_2$-rich light hydrocarbons, recycled $H_2$-rich light hydrocarbons and/or any other compounds which are desired to be added to the isomerization reaction zone. The term "$H_2$-rich light hydrocarbons" as used herein means a process stream containing at least 50 mole % $H_2$ with the balance being $C_1$-$C_3$ hydrocarbons. Recycled $H_2$-rich light hydrocarbons are commonly obtained from the product separation facilities of the isomerization process.

The sulfur level of the combined feed can exceed the preferred maximum level of 200 wt. ppm when sulfur build-up occurs. This is a result of recycling $H_2$-rich light hydrocarbons containing measurable quantities of sulfur, typically in the form of $H_2S$. The $H_2S$ is formed when sulfur compounds associated with the isomerizable hydrocarbons are converted in the isomerization reaction zone. Consequently, the $H_2S$, because of its high volatility, is separated concurrently with the $H_2$-rich light hydrocarbons in the product separation facilities. As more and more sulfur compounds are converted to $H_2S$ in the reaction zone the level of $H_2S$ in the recycle stream will constantly increase to an equilibrium level consistent with product separator operation conditions. Thus, as the recycle stream is admixed with the other process streams making up the combined feed, the total sulfur content of the combined feed will increase to a level 20 to about 80% greater than the fresh feed sulfur level depending on the separator temperature and pressure.

Accordingly, an embodiment of the present invention is the control of the combined feed sulfur level within the range of from about 5 to 150 wt. ppm. Any control means known in the art can be used, including catalytic conversion, physical or chemical adsorption, physical separation and the like. One preferred method of control involves having no recycle of $H_2$-rich light hydrocarbons from a product separation facility. This scheme would prevent the occurrence of sulfur build-up as described above. Another preferred method to control the sulfur level of the combined feed is by subjecting recycled $H_2$-rich light hydrocarbons to a sulfur removal process prior to addition to the combined feed. This removal process may reduce or eliminate the sulfur compounds from the recycled $H_2$-rich light hydrocarbons by any means known in the art including adsorption processes, catalytic processes, or combinations thereof. Adsorption processes may employ molecular sieves, high surface area silica-aluminas, carbon molecular sieves, crystalline aluminosilicates, activated carbons, and the like. Catalytic processes may employ traditional sulfur reducing catalyst formulations known to the art including refractory inorganic oxide supports containing metals selected from the group comprising Group VIB, Group IIB, and Group VIII metals. The amount of sulfur control can be varied to obtain the desired sulfur level in the combined feed. Low levels of sulfur in the fresh isomerizable hydrocarbons (5-150 ppm) requires no sulfur removal from the recycled $H_2$-rich light hydrocarbons as sufficiently low levels of recycle $H_2S$ (§ 100 ppm) can be maintained via losses of $H_2S$ in the product separator liquid and conversely a high level of sulfur in the fresh isomerizable hydrocarbon (§ 150 ppm) may require sulfur removal from the recycled $H_2$-rich light hydrocarbons.

The following examples are presented for purposes of illustration only and are not intended to limit the scope of the present invention.

A number of experiments were conducted to study how changes in the surface area of isomerization catalyst composites affect isomerization performance. Five catalysts were prepared for evaluation. In all the catalyst preparations, described in the following examples, the starting material was the hydrogen form, low sodium, partially dealuminated synthetic mordenite powder (marketed by Union Carbide under the name LZ-M-8), hereinafter referred to as the as received mordenite.

## EXAMPLE I

In this example, the method of formulating the catalyst, designated as Catalyst A, was not in accordance with the subject invention. A 9:1 weight ratio mixture of as received mordenite and alumina was admixed with an acidified peptization solution and extruded by means known in the art. The extruded composite was dried, calcined in an oxidative atmosphere, impregnated with platinum, and calcined again. The platinum was added to a level of 0.324 wt. %, based on the weight of the finished catalyst. A surface area of 567 $m^2/g$ was measured

for Catalyst A and correlated against its isomerization performance. This correlation is shown graphically in the attached Figure 1.

## EXAMPLE II

The catalyst composite of this example was designated at Catalyst B and was also not made in accordance with the subject invention. The as received mordenite powder was contacted with an acidic aqueous solution containing ammonium ions comprising 10 wt. % HCl and 10 wt. % $NH_4Cl$ at 140°F (60°C) for 150 minutes at a solution to zeolite weight ratio of 5:1. The resulting mordenite powder was washed with $H_2O$ and calcined prior to admixture with alumina and peptization solution. Extrusion and platinum addition were performed in an equivalent manner for Catalyst B as for Catalyst A of Example I. The platinum level of Catalyst B was 0.321 wt. %. A surface area of 534 $m^2/g$ was measured for Catalyst B and correlated against isomerization performance as shown in the attached Figure 1.

## EXAMPLE III

Catalyst C made in accordance with the invention was formulated substantially in the same manner as Catalyst A of Example I. However, in accordance with the subject invention, the dried extrudate prior to calcining and platinum addition was contacted with an acidic aqueous solution containing ammonium ions. This solution contained 10 wt. % HCl and 10 wt. % $NH_4Cl$. Contacting of the solution and the extrudate was performed at 140°F(60°C) for 120 minutes at a solution to zeolite weight ratio of 25:1. The extrudate was subsequently dried, calcined and treated with platinum following the same procedures used for Catalysts A and B. Catalyst C had a platinum level of 0.396 wt. %. A surface area of 622 $m^2/g$ was measured for Catalyst C and correlated against its isomerization performance as shown in the attached Figure 1.

## EXAMPLE IV

The calcined extruded composite of Example II, prior to platinum addition, was subjected to a second contact with an acidic aqueous solution in the manner as was Catalyst C in Example III. This second contact of the formed composite with the acidic aqueous solution is in accordance with the subject invention. Platinum was then added to the composite to a level of 0.308 wt. % and designated as Catalyst D. A surface area of 630 $m^2/g$ was measured for Catalyst D and correlated against its isomerization. This correlation is illustrated in the accompanying Figure 1.

## EXAMPLE V

Catalyst E was formulated in the same manner as Catalyst A except that the acidity of the peptization solution was reduced by 90%. As in Example I, Catalyst E was not formulated in accordance with the present invention. Catalyst E had a platinum level of 0.313 wt. %. A surface area of 542 $m^2/g$ was measured for Catalyst E was measured and correlated against its isomerization performance test results. This correlation is shown in the attached Figure 1.

## EXAMPLE VI

The five catalyst formulations of Examples I through V consisting of platinum supported on a hydrogen form mordenite gamma-alumina composite were evaluated for isomerization performance in a flow reactor processing a feed comprising a mixture of 46 wt. % n-pentane, 47.0 wt. % n-hexane, 5.5 wt.% methyl cyclopentane and 1.5 wt. % benzene.

The operating conditions used to test the isomerization performance of Catalysts A through E comprised a reactor pressure of 21.7 bar (315 psia), a liquid hourly space velocity of 1.0 $hr^{-1}$, a $H_2$ to feed hydrocarbon molar ratio of 1.0 and temperatures ranging between 254°C (490°F) and 277°C (530°F). Test run data, specifically the selectivities to isopentane (i-$C_5$ and to 2,2-dimethyl butane (2,2 DMB) at 97 wt. % $C_5^+$ yield, were used as measures of isomerization performance. The selectivity value for i-$C_5$ presented in the accompanying Figure 1 is defined as the weight fraction of the i-$C_5$ in the liquid isomerization product divided by the weight fraction of the total amount of $C_5$ hydrocarbon in the product. To obtain the 2,2 selectivity value shown in Figure 1, the weight fraction of 2,2 DMB in the liquid isomerization product is divided by the total, non-cyclic, $C_5$ hydrocarbon in the product.

At the isomerization test run conditions utilized in the above examples, the selectivities to i-$C_5$ and 2,2 DMB are a direct function of the measured surface area of the finished catalyst. As graphically illustrated in the attached Figure 1, the selectivities to i-$C_5$ and 2,2 DMB increase as the measured surface area increases. Catalyst C of Example III and Catalyst D of Example IV demonstrate the highest selectivities to i-$C_5$ and 2,2 DMB in accordance with the invention, both having measured surface areas of at least 580 $m^2/g$. Comparison of the platinum levels of Catalysts C and D to each other and to the other three catalysts shows that the platinum level does not correlate with isomerization performance further substantiating the benefit of having catalyst composites with surface areas of at least 580 m²/g. Thus, utilizing the catalytic composition of the present invention clearly yields catalysts with superior isomerization performance.

## EXAMPLE VII

Two isomerization process tests were conducted to study how sulfur containing feedstocks affect isomerization performance. The first process, designated as Process A, utilized an isomerization catalyst having a surface area not in accordance with the subject invention. Process A was tested to demonstrate the isomerization performance when a sulfur containing feed is subjected to a conventional isomerization catalyst of the prior art. The catalyst used was identical to Catalyst A of Example I.

The second process, designated as Process B, was in accordance with the present invention and utilized Catalyst C that was formulated in Example IV in accordance with the subject invention.

Processes A and B were evaluated for isomerization performance in a flow reactor processing a feed comprising a mixture of 6.8 wt. % butane, 20.9 wt. % n-pentane, 14.5 wt. % i-pentane, 15.7 wt. % n-hexane, 19.0 wt. % i-hexane, 12.4 wt. % cyclopentanes/cyclohexanes and 2.5 wt. % benzene, 8.2 wt. % $C_6^+$, and 133 wt. ppm sulfur.

The operating conditions used to test the isomerization performance of Processes A and B comprised a reactor pressure of 32.0 bar (450 psig), a liquid hourly space velocity of 1.0 hr $^{-1}$, a $H_2$ to feed hydrocarbon molar ratio of 2.0 and temperatures ranging between 254°C (490°F) and 316°C (600°F). The Research octane value (RON-O) of the $C_5^+$ liquid product, the average reactor catalyst bed temperature, and the weight percent $C_5^+$ yield were used to determine isomerization performance. The RON-O is shown in the attached Figures 2 and 3 as a function of both the average reactor bed temperature and the $C_5^+$ yield. The process of the instant invention, Process B, has a significant activity advantage over the conventional isomerization process, Process A, employing a prior art catalyst. This is illustrated in Figure 2 by the 32.2°C (58°F) lower temperature requirement to achieve 78 RON-O. Process B also exhibits a significant octane advantage of up to four numbers at 94 wt. % $C_5^+$ yield, compared to Process A, as shown in Figure 2.

These examples clearly demonstrate the superior performance of the subject invention when compared to the isomerization performance obtained with a conventional process employing a prior art catalyst. A comparison at equivalent product yields shows the invention of the instant application to have higher isomerization activity with an increased product octane value. Conversely, a comparison at equivalent product octane (e.g., 78 RON) shows the invention of the instant application to have a greater product yield.

## Claims

1. A process for isomerizing isomerizable hydrocarbons by contacting a feed stream containing said isomerizable hydrocarbons and a hydrogen stream with a catalyst at isomerization conditions characterized in that the catalyst comprises a combination of a platinum group metal component with a support containing hydrogen-form crystalline mordenite and a refractory inorganic oxide, having a surface area of at least 580 m²/g.

2. A process according to Claim 1 characterized in that the platinum group metal component is platinum and it is present in an amount of from 0.1 to 5.0 wt. percent of the catalyst composite.

3. A process according to Claim 2 characterized in that the amount of platinum is from 0.15 to 0.5 wt. percent.

4. A process according to any one of the preceding Claims characterized in that the hydrogen-form crystalline mordenite has a silica to alumina ratio of at least 16:1.

5. A process according to any one of the preceding Claims characterized in that crystalline mordenite is present in an amount of from 75 to 95 percent by weight of the composite.

6. A process according to any one of the preceding Claims characterized in that the refractory inorganic oxide is gamma-alumina, eta-alumina, or a mixture thereof.

7. A process according to any one of the preceding Claims characterized in that the catalyst comprises a catalytic amount of a promoter metal.

8. A process according to any one of the preceding Claims characterized in that the isomerizable hydrocarbons comprise normal paraffins having from 4 to 7 carbon atoms per molecule.

9. A process according to any one of the preceding Claims characterized in that the isomerization conditions comprise a temperature of from 93 to 427°C, a pressure of from 690 to 6895 KPa gage, a liquid hourly space velocity of from 0.25 to 5 hr.$^{-1}$ and a hydrocarbon mole ratio of 0.5 to 5 mole of $H_2$/mole of isomerizable hydrocarbon.

10. A process according to any one of the preceding Claims characterized in that the combination of the hydrocarbon feed stream and the hydrogen stream contains sulfur compounds in an amount equivalent to 5 to 200 wt. ppm of the combination, calculated on an elemental sulfur basis.

11. A process according to Claim 10 characterized in that the amount of sulfur charged to the process is controlled by having no recycle of $H_2$-rich light hydrocarbons from a product separation facility.

12. A process according to Claim 10 characterized in that the amount of sulfur charged to the process is controlled by subjecting a recycled $H_2$-rich light hydrocarbon stream from a product separation facility to a sulfur removal process prior to charging to the process.

13. A method of manufacturing a hydrocarbon isomerization catalyst composition characterized by the steps of:
(a) forming a composite of hydrogen-form mordenite and a refractory inorganic oxide;
(b) calcining said composite;
(c) contacting said composite at a temperature of 25 to 100°C for a period of 0.5 to 4 hours with an acidic aqueous solution having an acid concentration from 0.5 to 6M, and containing ammonium ions at a concentration of 0.5 to 4M, whereby to provide a surface area of at least 580 m²/g.;
(d) calcining said contacted composite; and
(e) incorporating the platinum group metal component into the calcined composite formed by step (d).

**Revendications**

1. Procédé d'isomérisation d'hydrocarbures isomérisables, en mettant en contact un courant d'alimentation contenant ces hydrocarbures isomérisables, et un courant d'hydrogène, avec un catalyseur dans des conditions d'isomérisation, caractérisé en ce que le catalyseur comprend un composant dérivé de métal du groupe du platine associé à un support contenant de la mordénite cristalline sous forme hydrogénée et un oxyde inorganique réfractaire, ayant une surface spécifique d'au moins 580 m²/g.

2. Procédé selon la revendication 1, caractérisé en ce que le composant dérivé de métal du groupe du platine, est le platine, et en ce qu'il est présent selon une quantité de 0,1 à 5,0 % en poids par rapport à la composition catalytique.

3. Procédé selon la revendication 2, caractérisé en ce que la quantité de platine, est de 0,15 à 0,5 % en poids.

4. Procédé selon l'une quelconque des revendications précédentes, caractérisé en ce que la mordénite cristalline sous forme hydrogénée, a un rapport de la silice à l'alumine, d'au moins 16:1.

5. Procédé selon l'une quelconque des revendications précédentes, caractérisé en ce que la mordénite cristalline est présente selon une quantité de 75 à 95 % en poids par rapport à la composition.

6. Procédé selon l'une quelconque des revendications précédentes, caractérisé en ce que l'oxyde inorganique réfractaire, est l'alumine gamma, l'alumine éta, ou des mélanges de celles-ci.

7. Procédé selon l'une quelconque des revendications précédentes, caractérisé en ce que le catalyseur comprend une quantité catalytique d'un métal promoteur.

8. Procédé selon l'une quelconque des revendications précédentes, caractérisé en ce que les hydrocarbures isomérisables comprennent les paraffines normales comportant de 4 à 7 atomes de carbone par molécule.

9. Procédé selon l'une quelconque des revendications précédentes, caractérisé en ce que les conditions d'isomérisation comprennent une température de 93 à 427 °C, une pression relative de 690 à 6 895 kPa, une vitesse spatiale horaire du liquide de 0,25 à 5 h$^{-1}$ et une proportion molaire des hydrocarbures, de 0,5 à 5 moles de $H_2$ par mole d'hydrocarbure isomérisable.

10. Procédé selon l'une quelconque des revendications précédentes, caractérisé en ce que le mélange du courant d'alimentation hydrocarboné et du courant d'hydrogène, contient des composés sulfurés selon une quantité équivalente à 5 jusqu'à 200 ppm en poids par rapport au mélange, d'après calcul par rapport au soufre élémentaire.

11. Procédé selon la revendication 10, caractérisé en ce que la quantité de soufre introduite dans le cycle du procédé, est ajustée en ne recyclant pas les hydrocarbures légers riches en $H_2$ à partir d'un dispositif de séparation de produit.

12. Procédé selon la revendication 10, caractérisé en ce que la quantité de soufre introduite dans le cycle du procédé, est ajustée en soumettant un courant d'hydrocarbures légers riches en $H_2$ issu d'un dispositif de séparation de produit, à un traitement d'élimination du soufre, avant de l'introduire dans le cycle du procédé.

13. Procédé de préparation d'une composition catalytique pour l'isomérisation d'hydrocarbures, caractérisé en ce qu'il comprend les étapes consistant :

(a) à former une composition de mordénite sous forme hydrogénée et d'un oxyde inorganique réfractaire ;

(b) à calciner cette composition ;

(c) à mettre en contact cette composition, à une température de 25 à 100 °C, pendant 0,5 à 4 heures, avec une solution aqueuse acide ayant une concentration en acier de 0,5 à 6 M, et contenant des ions ammonium selon une concentration de 0,5 à 4 M, de façon à obtenir une surface spécifique d'au moins 580 $m^2/g$ ;

(d) à calciner cette composition obtenue d'après la mise en contact ; et

(e) à incorporer le composant dérivé de métal du groupe du platine dans la composition calcinée obtenue dans l'étape (d).

## Patentansprüche

1. Verfahren zum Isomerisieren von isomerisierbaren Kohlenwasserstoffen durch Inkontaktbringen eines die genannten isomerisierbaren Kohlenwasserstoffe und einen Wasserstoffstrom enthaltenden Zulaufs mit einem Katalysator unter Isomerisierungsbedingungen, dadurch gekennzeichnet, daß der Katalysator eine Kombination einer Komponente aus der Gruppe der Platinmetalle mit einem Träger umfaßt, der eine Oberflächengroße von mindestens 580 $m^2/g$ besitzt und aus kristallinem Mordenit in Wasserstofform und einem feuerfesten organischen Oxid besteht.

2. Verfahren nach Anspruch 1, dadurch gekennzeichnet, daß es sich bei der Komponente aus der Gruppe der Platinmetalle um Platin handelt und daß es in einer Menge von 0,1 bis 5,0 Gew.% der Katalysatorzusammensetzung vorliegt.

3. Verfahren nach Anspruch 2, dadurch gekennzeichnet, daß die Platinmenge 0,15 bis 0,5 Gew.% beträgt.

4. Verfahren nach einem der vorhergehenden Ansprüche, dadurch gekennzeichnet, daß das kristalline Mordenit in Wasserstofform ein Verhältnis Siliciumdioxid zu Aluminiumoxid von mindestens 16:1 hat.

5. Verfahren nach einem der vorhergehenden Ansprüche, dadurch gekennzeichnet, daß das kristalline Mordenit in einer Menge von 75 bis 95 Gew.% der Zusammensetzung vorliegt.

6. Verfahren nach einem der vorhergehenden Ansprüche, dadurch gekennzeichnet, daß es sich bei dem feuerfesten anorganischen Oxid um Gamma-Aluminiumoxid, Eta-Aluminiumoxid oder ein Gemisch beider handelt.

7. Verfahren nach einem der vorhergehenden Ansprüche, dadurch gekennzeichnet, daß der Katalysator eine katalytische Menge eines Promotormetalls enthält.

8. Verfahren nach einem der vorhergehenden Ansprüche, dadurch gekennzeichnet, daß die isomerisierbaren Kohlenwasserstoffe normale Paraffine mit 4 bis 7 Kohlenstoffatomen je Molekül enthalten.

9. Verfahren nach einem der vorhergehenden Ansprüche, dadurch gekennzeichnet, daß die Isomerisierungsbedingungen im Bereich einer Temperatur von 93 bis 427 °C, eines Drucks von 690 bis 6895 kPa, einer Raumgeschwindigkeit von 0,25 bis 5 $h^{-1}$ und eines Kohlenwasserstoff-Molverhältnisses von 0,5 bis 5 Mol $H_2$/Mol isomerisierbarem Kohlenwasserstoff liegen.

10. Verfahren nach einem der vorhergehenden Ansprüche, dadurch gekennzeichnet, daß die Kombination des Kohlenwasserstoffzulaufs und Wasserstoffzulaufs Schwefelkomponenten in einer äquivalenten Menge von 5 bis 200 Gew.-ppm der Kombination, bezogen auf elementaren Schwefel, enthält.

11. Verfahren nach Anspruch 10, dadurch gekennzeichnet, daß die Menge des in das Verfahren eingebrachten Schwefels in der Weise überwacht wird, daß keine Rückführung von $H_2$-reichen leichten Kohlenwasserstoffen aus einer Produkttrennanlage erfolgt.

12. Verfahren nach Anspruch 10, dadurch gekennzeichnet, daß die in das Verfahren eingeführte Schwefelmenge in der Weise überwacht wird, daß ein Strom $H_2$-reicher leichter Kohlenwasserstoffe aus einer Produkttrennanlage vor dem Eintritt in das Verfahren einen Entschwefelungsprozeß durchläuft.

13. Verfahren zum Herstellen einer Katalysatorzusammensetzung für die Kohlenwasserstoffisomerisierung, gekennzeichnet durch folgende Schritte:

　　(a) Herstellen einer Zusammensetzung von Mordenit in Wasserstofform und einem feuerfesten anorganischen Oxid;

　　(b) Kalzinieren dieser Zusammensetzung;

　　(c) Inkontaktbringen dieser Zusammensetzung bei einer Temperatur von 25 bis 100°C für eine Dauer von 0,5 bis 4 h mit einer wäßrigen sauren Lösung, die eine Säurekonzentration von 0,5 bis 6 M aufweist und Ammoniumionen in einer Konzentration von 0,5 bis 4 M enthält, wodurch eine Oberflächengröße von mindestens 580 m²/g entsteht;

　　(d) Kalzinieren der genannten kontaktierten Zusammensetzung; und

　　(e) Einarbeiten der Komponente aus der Gruppe der Platinmetalle in die in Schritt (d) gebildete kalzinierte Zusammensetzung.

Figure 1

Selectivity For $i-C_5$ And 2,2 DMB
Vs.
Langmuir Surface Area

Legend: O——O Catalyst – A; □——□ Catalyst – B; △——△ Catalyst – C; X——X Catalyst – D; ▽——▽ Catalyst – E

Langmuir Surface Area, $m^2/g$.

## Figure 2

| O —— O — Process — A |
| Δ —— Δ — Process — B |

$C_5^+$ Research Octane vs. Reactor Temperature

Average Rx Bed Temperature, °F.

## Figure 3

$C_5^+$ Research Octane vs. $C_5^+$ Product Yield

$C_5^+$ Yield, Wt. %

13